# DEMANDE DE BREVET EUROPEEN

(11) **EP 4 772 223 A1**
(43) Date de publication de la demande: **08.07.2026**
(21) Numéro de dépôt: 25222224.5
(22) Date de dépôt: 10.12.2025
(51) Int. Cl.: A61M 16/08, A61M 16/12

(54) **INSTALLATION D'ADMINISTRATION DE NO GAZEUX AVEC MODULE D'INJECTION DE GAZ**

(30) Priorité: 06.01.2025 FR 2500093
(71) Demandeur: INOSYSTEMS, 92160 Antony (FR)
(72) Inventeur: BOULANGER, Thierry, 92160 Antony (FR); PATEL, Akshar, 92160 Antony (FR); MA, Jiefu, 92160 Antony (FR); MARCHAL, Frederic, 92160 Antony (FR)
(74) Mandataire: Air Liquide

(57) **Abrégé**

L'invention concerne une installation de fourniture de gaz comprenant un module d'injection de gaz, auquel sont raccordés un ventilateur mécanique et un appareil de délivrance de NO. Le module d'injection de gaz (500, 800) comprend un corps de module tubulaire (803) traversé par un passage interne (811) d'axe longitudinal (AA) et comprenant: un port amont (801); un port aval (802); un conduit d'injection (807) externe comprenant un passage interne (813) pour véhiculer un flux gazeux de NO/N₂ ; et une expansion tubulaire (812) formant buse d'injection et comprenant un port d'injection (809) agencé à une extrémité libre et débouchant dans le passage interne du corps de module tubulaire. L'expansion tubulaire a une forme générale courbe ou coudée, et comprend un tronçon portant le port d'injection (809), agencé parallèlement à l'axe (AA).

## Description

L'invention concerne une installation de fourniture de gaz à base de NO à une personne en ayant besoin à des fins d'assistance de ses fonctions respiratoires, typiquement un patient, comprenant un module d'injection de gaz amélioré agencé dans le circuit patient de l'installation acheminant un flux de gaz respiratoire contenant de l'oxygène (>20%vol.) provenant d'un ventilateur médical, tel un mélange N₂/O₂ ou de l'air, ledit module d'injection étant configuré pour y raccorder par ailleurs un appareil de délivrance de NO fournissant un flux gazeux contenant du NO, de manière à pouvoir réaliser un mélange homogène de ces gaz au moyen dudit module d'injection et à obtenir ainsi un mélange gazeux combiné contenant du NO à une posologie donnée, qui est ensuite administré par voie inhalée au patient en ayant besoin.

Le monoxyde d'azote ou « NO » est un gaz qui, lorsqu'il est inhalé, dilate les vaisseaux sanguins des poumons, ce qui permet d'augmenter l'oxygénation du sang en favorisant les échanges gazeux. Cette propriété est exploitée pour traiter l'hypertension artérielle pulmonaire chez l'adulte, l'enfant ou les nouveau-nés.

Habituellement, le NO, typiquement un mélange de NO et d'azote (N₂), est ajouté au gaz respiratoire non-hypoxique, qui est inhalé par le patient souffrant d'hypertension pulmonaire selon une posologie déterminée, généralement fixée par un médecin, qui varie généralement entre 1 et 100 ppm en volume, typiquement entre 1 et 40 ppm vol.

Pour ce faire, le mélange NO/N₂ est fourni par un appareil de délivrance de NO qui vient se raccorder fluidiquement à un module d'injection de NO qui est agencé dans la branche inspiratoire d'un circuit patient d'une installation NO installation de fourniture de gaz, i.e. d'un mélange gazeux à base de NO, alimentant le patient à traiter.

Le circuit patient permet de relier fluidiquement un ventilateur médical, c'est-à-dire un appareil d'assistance respiratoire, fournissant le gaz respiratoire contenant au moins 20 à 21%vol. d'oxygène, tel de l'air ou un mélange O₂/N₂, à une interface respiratoire alimentant le patient à traiter, tel un masque respiratoire, une sonde trachéale ou analogue, comme enseigné par US5558083 ou EP4209243.

Le flux de gaz respiratoire (e.g. air ou O₂/N₂) provenant du ventilateur médical est donc véhiculé par le circuit patient, traverse le module d'injection de NO où s'effectue son mélange avec le mélange NO/N₂, et le mélange gazeux combiné sortant du module d'injection de NO chemine dans le circuit patient jusqu'à l'interface respiratoire fournissant le mélange combiné contenant le NO au patient.

Autrement dit, le module d'injection de NO permet d'injecter le mélange NO/N₂ provenant de l'appareil de délivrance de NO, dans le flux de gaz respiratoire provenant du ventilateur médical, et ainsi obtenir le mélange gazeux combiné ou final contenant au moins N₂, O₂ (>20%vol) et du NO à la posologie souhaitée.

La quantité de mélange NO/N₂, c'est-à-dire le débit de NO/N₂ à injecter dans le flux de gaz respiratoire, via le module d'injection de NO, est calculée à partir de mesures de débit opérées par un capteur de débit agencé dans le circuit patient entre le ventilateur médical et ledit module d'injection de NO. Le capteur de débit permet mesurer le débit du flux gazeux respiratoire délivré par le ventilateur médical et de retourner les mesures à l'appareil de délivrance de NO pour que celui-ci en déduise, i.e. calcule, le débit de NO à délivrer, i.e. de mélange gazeux NO/N₂, en relation avec la posologie désirée. Le capteur de débit peut être associé ou intégré au module d'injection de NO.

En pratique, il existe un problème résultant de l'injection du flux de NO, via le module d'injection de NO, dans le gaz respiratoire contenant de l'oxygène provenant du ventilateur médical, à savoir une oxydation excessive pouvant se produire, au sein du module d'injection de NO, du NO par de l'oxygène (O₂) présent dans le flux de gaz respiratoire, avec formation d'espèces dioxyde d'azote (NO₂) toxiques se retrouvant dans le mélange gazeux combiné envoyé au patient.

Si cette oxydation intempestive du NO avec formation de NO₂ dépend des teneurs en NO et O₂, elle est d'autant plus importante que la teneur en NO du mélange NO/N₂ est élevée. En effet, la formation de NO₂ dépend de la concentration initiale en NO au carré, alors qu'elle est linéaire avec la concentration en O₂. Autrement dit, la concentration initiale en NO a un impact bien supérieur à celle en O₂ sur la formation de NO₂.

Or, les modules d'injection de NO actuels ont été conçus pour des concentration en NO initiales dans le mélange NO/N₂ inférieures à 800 ppmv de NO et dès lors, ne permettent pas de garantir une concentration en NO₂ acceptable, lorsque la concentration en NO est plus élevée, par exemple de plusieurs milliers de ppmv. A titre indicatif, passer d'une teneur en NO de 800 ppmv à une teneur de 5000 ppmv dans le mélange NO/N₂ fourni par l'appareil de délivrance de NO conduit à multiplier la concentration en NO par 6.25, ce qui engendre une formation de NO₂ 39 fois supérieure. De façon analogue, passer d'une teneur en NO de 800 ppmv à 20000 ppmv conduit à une formation de NO₂ 625 fois supérieure.

Il convient dès lors de pouvoir obtenir le plus rapidement possible un mélange gazeux combiné homogène à la posologie en NO désirée, par exemple 20 ppmv de NO, car lorsqu'il est homogène, l'oxydation du NO est plus lente et la formation de NO₂ inférieure, et ce, même avec des teneurs élevées en NO, typiquement de plusieurs milliers de ppmv.

A cette fin, US10894140 propose d'utiliser un module d'injection de NO à port d'injection unique ou multiple, i.e. un ou plusieurs entrées de NO, en considérant un ratio à ne pas dépasser en termes de rapport de vélocité de gaz, c'est-à-dire de rapport entre la vélocité du gaz respiratoire (O₂>20%vol.) issu du ventilateur médical et celle du gaz à base de NO issu du dispositif de délivrance de NO (i.e. mélange N₂/NO). Dans les deux cas, l'injection se fait de manière transverse où les deux gaz viennent se mélanger dans le module d'injection en étant dirigés/orientés de façon perpendiculaire l'un à l'autre.

Cependant, utiliser un module d'injection de NO conçu pour opérer une injection transverse n'est pas idéal. En effet, il a été constaté que de l'O₂ présent dans le flux issu du débit du ventilateur, peut remonter par diffusion dans la ligne d'acheminement de gaz reliant l'appareil de délivrance de NO au module d'injection de NO, lors du fonctionnement de l'installation, et générer ainsi une quantité de NO₂ additionnelle qui va se retrouver ensuite dans le mélange combiné sortant du module d'injection de NO. De plus, il a été aussi observé qu'opérer une injection transverse de NO peut créer localement un déséquilibre de concentrations et conduire à une formation excessive de NO₂. Ces formations d'espèces NO₂ additionnelles ne sont pas souhaitables, ni acceptables, et ce, d'autant moins que la concentration en NO dans le flux d'alimentation (i.e. NO/N₂) est élevée.

D'une façon générale, du fait de la formation accélérée de NO₂ en fonction de la concentration en NO de la source N₂/NO, il apparait important de limiter encore plus la formation de NO₂, en particulier pour des concentrations importantes de la source N₂/NO.

Un but de l'invention est dès lors de proposer une installation de fourniture de gaz à base de NO comprenant un circuit patient, dans lequel est agencé un module d'injection de NO amélioré permettant de minimiser la formation de NO₂ toxiques par oxydation du NO, tout en conduisant à la formation d'un mélange gazeux combiné homogène, après injection, grâce à ce module, d'un flux gazeux à base NO (e.g. NO/N₂) provenant d'un appareil de délivrance de NO, dans un flux de gaz respiratoire contenant de l'oxygène (e.g. air ou O₂/N₂) provenant d'un ventilateur médical, et ce, de préférence, même lorsque la teneur en NO dans le flux de NO (e.g. NO/N₂) est élevée, c'est-à-dire typiquement de plusieurs milliers de ppmv.

Une solution selon l'invention concerne une installation de fourniture de gaz, typiquement un mélange gazeux combiné contenant du NO et de l'oxygène, comprenant un module d'injection de gaz, auquel sont raccordés un ventilateur mécanique fournissant un flux de gaz respiratoire contenant au moins 20%vol. d'oxygène, et un appareil de délivrance de NO fournissant un flux de mélange gazeux NO/N₂.

Le module d'injection de gaz comprend un corps de module tubulaire traversé par un passage interne (i.e. lumen) comprenant un axe longitudinal (AA).

Le corps de module tubulaire comprend en outre :
- un port amont (i.e. orifice d'entrée ou analogue) par lequel le flux de gaz respiratoire peut entrer dans le passage interne,
- un port aval (i.e. orifice de sortie ou analogue) par lequel un flux gazeux combiné peut ressortir du passage interne,
- un conduit d'injection externe venant se raccorder fluidiquement au corps de module tubulaire, et comprenant un passage interne pour véhiculer le flux gazeux de NO/N₂, et
- une expansion tubulaire formant une buse d'injection agencée dans le passage interne du corps tubulaire et comprenant un passage de buse interne en communication fluidique avec le passage interne du conduit d'injection, ladite expansion tubulaire comprenant en outre un port d'injection agencé à une extrémité libre de ladite expansion tubulaire, ledit port d'injection débouchant dans le passage interne du corps de module tubulaire.

De plus, dans le module d'injection de gaz de l'installation de l'invention, l'expansion tubulaire a une forme générale courbe ou coudée, c'est-à-dire qu'elle forme une « buse courbée », et comprend au moins un tronçon portant le port d'injection, agencé parallèlement à l'axe longitudinal (AA) et dirigé (i.e. orienté) en direction du port aval.

Ainsi, grâce au module d'injection de gaz à buse courbée utilisé dans l'installation selon l'invention, on opère une injection du mélange NO/N₂, dans le passage interne (i.e. lumen) d'axe longitudinal (AA) du module, dans le même sens, i.e. la même direction, c'est-à-dire selon l'axe AA, que le flux de gaz respiratoire contenant au moins 20%vol. d'oxygène, entrant par le port amont et circulant dans le passage interne (lumen) du corps tubulaire en direction du port aval par lequel ressort le flux gazeux combiné, i.e. le flux gazeux contenant du NO et de l'oxygène.

Selon le mode de réalisation considéré, le module d'injection de gaz de l'installation selon l'invention peut comprendre l'une ou plusieurs des caractéristiques suivantes :
- le port amont du module d'injection de gaz est raccordé fluidiquement au ventilateur mécanique fournissant le flux de gaz respiratoire, de préférence par l'intermédiaire d'au moins une partie de la branche inspiratoire du circuit patient.
- le conduit d'injection externe est raccordé fluidiquement à l'appareil, de délivrance de NO de préférence par l'intermédiaire d'un conduit de gaz, tel un tuyau flexible.
- le passage interne comprend une chambre amont comprenant le port amont, c'est-à-dire que le port amont communique fluidiquement avec la chambre amont.
- le passage interne comprend une chambre de mélange aval comprenant le port aval, c'est-à-dire que le port aval communique fluidiquement avec la chambre de mélange aval.
- le corps de module tubulaire est préférentiellement cylindrique ou analogue.
- l'expansion tubulaire a une forme générale en « L » ou analogue.
- le tronçon portant le port d'injection de l'expansion tubulaire est agencé coaxialement à l'axe longitudinal (AA) du passage interne du corps de module tubulaire.
- l'expansion tubulaire comprend un premier tronçon de conduit et un second tronçon de conduit.
- le second tronçon de conduit comprend le port d'injection et l'extrémité libre de ladite expansion tubulaire.
- le premier tronçon de conduit est agencé entre le second tronçon de conduit et le passage interne du conduit d'injection et en communication fluidique avec le second tronçon de conduit et le passage interne.
- le conduit d'injection externe vient se raccorder à la paroi périphérique tubulaire du corps de module tubulaire.
- le corps de module tubulaire est préférentiellement en polymère, typiquement en polycarbonate ou en polysulfone par exemple.
- l'expansion tubulaire est formée d'une pièce, par exemple par moulage par injection ou toute autre technique adaptée.
- l'expansion tubulaire constitue une buse d'injection coudée, c'est-à-dire courbe ou recourbée.
- le corps de module tubulaire est configuré, au niveau du port amont et du port aval, pour permettre un raccordement fluidique de conduits de gaz formant au moins une partie de la branche inspiratoire du circuit patient, typiquement d'un conduit de gaz amont et un conduit de gaz aval, tels des tuyaux flexibles.
- le corps de module tubulaire comprend des moyens de raccordement de conduit permettant d'y raccorder fluidiquement les conduit de gaz amont et aval de la branche inspiratoire du circuit patient.
- les moyens de raccordement de conduit comprennent des raccords ou analogues, par exemple des raccords à emboitement, vissés, à baïonnette ou analogues.
- le conduit de gaz amont constitue une partie amont de la branche inspiratoire du circuit patient servant à relier fluidiquement le ventilateur médical au module d'injection de gaz afin de l'alimenter en gaz respiratoire contenant au moins 20%vol. d'O₂.
- le circuit patient alimente une interface respiratoire équipant le patient en mélange gazeux combiné.
- l'interface respiratoire comprend un masque respiratoire, une sonde d'intubation trachéale ou autre dispositif respiratoire adapté.
- le conduit de gaz aval constitue une partie aval de la branche inspiratoire du circuit patient servant à recueillir et convoyer le mélange gazeux combiné formé au sein du corps de module tubulaire, c'est-à-dire de relier fluidiquement le module d'injection de gaz à l'interface respiratoire équipant le patient.
- l'expansion tubulaire du module d'injection de gaz est orientée pour opérer une injection du mélange NO/N₂, dans le passage interne d'axe longitudinal (AA) dans la même direction que le flux de gaz respiratoire contenant au moins 20%vol. d'oxygène, circulant dans le passage interne, c'est-à-dire dans le même sens de circulation que celui du flux de gaz respiratoire et selon l'axe (AA).

De plus, selon certains modes de réalisation, l'installation de fourniture de gaz selon l'invention peut comprendre aussi l'une ou plusieurs des caractéristiques suivantes :
- l'appareil de délivrance de NO est relié fluidiquement et alimenté en un mélange NO/N₂ par un ou plusieurs récipients de gaz contenant un mélange NO/N₂, telles des bouteilles de gaz.
- l'appareil de délivrance de NO comprend des moyens de pilotage, de préférence à microprocesseur, tel un contrôleur électronique ou analogue ; un circuit de gaz interne comprenant au moins une entrée de NO configurée pour y raccorder fluidiquement au moins un récipient de NO ; des moyens à vanne, telles des électrovannes, agencées sur le circuit de gaz interne pour contrôler le flux de NO/N₂ dans le circuit de gaz interne ; et au moins une sortie de NO pour fournir le flux gazeux de NO/N₂.
- des moyens de détente, tels un ou des dispositif détendeur de gaz, sont agencés en amont de l'appareil de délivrance de NO et configurés pour opérer une réduction de pression du gaz contenant du NO provenant du (ou des) récipient de NO jusqu'à une pression de détente (PD) donnée inférieure à 10 bar, typiquement de l'ordre de 4 à 8 bar, de préférence entre 4 et 7 bar.
- au moins une ligne d'amenée de gaz pour acheminer le gaz contenant du NO ayant traversé les moyens de détente jusqu'à ladite (ou aux) entrée de gaz de l'appareil de délivrance de NO, par exemple un ou plusieurs tuyaux flexibles ou similaires.
- lorsque le ou les récipients de NO sont pleins, le mélange NO/N₂ y est conditionné à une pression (i.e. une pression de départ) d'au moins 150 bar appelée « haute pression ». Cette haute pression correspond à la pression du gaz à base de NO avant détente, e.g. mélange NO/N₂, c'est-à-dire avant réduction de pression par passage dans les premiers et/ou les seconds moyens de réduction de pression.
- chaque récipient de gaz est équipé d'un robinet de distribution de gaz servant à contrôler la fourniture de gaz à base de NO provenant dudit récipient de gaz, c'est-à-dire la distribution de gaz.
- chaque récipient de NO contient un mélange NO/N₂ contenant entre 100 et 20000 ppmv.
- l'appareil de délivrance de NO est alimenté en un mélange NO/N₂ contenant entre 100 et 20 000 ppmv de NO, le reste étant de l'azote (N₂).
- l'appareil de délivrance de NO comprend des moyens de réglage de dose configurés pour permettre à un utilisateur de fixer ou sélectionner une consigne de teneur en NO correspondant à la proportion finale de NO souhaitée dans le mélange gazeux combiné, i.e. une posologie.
- les moyens de réglage de dose font partie d'une IHM (interface homme-machine) ou IGU (interface graphique utilisateur) de l'appareil de délivrance de NO.
- les moyens de réglage de dose de l'appareil comprennent une ou des touches tactiles, actionnables par l'utilisateur, s'affichant sur un écran digital à dalle tactile de l'IHM, i.e. un écran tactile.
- de préférence l'écran tactile est du type à affichage en couleurs.
- la consigne de teneur en NO est comprise entre 1 et 80 ppmv, typiquement entre 5 et 40 ppmv.
- les moyens de mémorisation de l'appareil de délivrance de comprennent une mémoire informatique, telle une mémoire flash, une RAM ou analogue.
- les moyens de pilotage comprennent un (ou des) (micro)processeur agencé sur une (ou des) carte électronique, typiquement un micro)contrôleur ou analogue.
- le (ou les) (micro)processeur mettent en oeuvre un ou des algorithmes, notamment un (ou des) algorithme de pilotage ou de contrôle des vannes ou valves, de traitement des mesures de débit ou de pression....
- les moyens de mémorisation sont agencés sur la carte électronique.
- l'appareil de délivrance de NO et le ventilateur mécanique sont chacun alimentés électriquement par une ou des sources de courant électrique, typiquement le secteur (110/220V) et/ou une ou des batteries rechargeables.
- le ventilateur mécanique est configuré pour fournir un flux de gaz respiratoire contenant de l'O₂ en une teneur d'au moins 20%vol., tel de l'air ou un mélange O₂/N₂, typiquement d'au moins 21%vol. environ.
- l'appareil de délivrance de NO et le ventilateur mécanique sont raccordés fluidiquement au module d'injection agencé sur/dans le circuit patient, en particulier sur/dans la branche inspiratoire du circuit patient.
- le module d'injection est configuré pour opérer un mélange du gaz contenant du flux à base de NO provenant de l'appareil de délivrance de NO (i.e. flux gazeux NO/N₂) avec le flux de gaz respiratoire contenant au moins 20%vol. d'O₂ fourni par le ventilateur, et obtenir un mélange gazeux combiné contenant (au moins) du NO et de l'oxygène.
- un capteur de débit est agencé dans le circuit patient entre le ventilateur médical et le dispositif d'injection, en particulier dans la branche inspiratoire du circuit patient.
- le module d'injection comprend une première entrée de gaz alimentée en flux de gaz respiratoire contenant de l'O₂ provenant du ventilateur médical ; et une seconde entrée de gaz alimentée en gaz contenant du NO provenant de l'appareil de délivrance de NO.
- le module d'injection comprend en outre une sortie de gaz combiné fournissant le mélange gazeux combiné contenant du NO et de l'oxygène, obtenu par mélange, au sein du module d'injection, du gaz contenant du NO (e.g. mélange NO/N₂) avec le flux de gaz respiratoire contenant de l'O₂ (e.g. air ou mélange O₂/N₂).
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- selon un mode de réalisation particulier, le ventilateur médical est de type HFO ou comprend une fonction de HFO, c'est-à-dire qu'il est apte à produire des oscillations à haute fréquence.
- chaque récipient de NO est ou comprend une (ou des) bouteille de gaz ayant une contenance comprise entre 0,5 et 50 L (équivalent en eau).
- chaque récipient de NO comprend un corps cylindrique en acier ou en alliage d'aluminium.
- le circuit patient de l'installation comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- le circuit patient, aussi appelé circuit respiratoire, en particulier la branche inspiratoire, peut comprendre un humidificateur de gaz, de préférence agencé en aval du module d'injection, de manière à pouvoir humidifier le mélange gazeux combiné avant son administration par inhalation au patient.

Selon encore un autre aspect, l'invention concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO assurant une délivrance de NO à la posologie souhaitée, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

Par ailleurs, l'invention porte aussi sur une utilisation d'un module d'injection de gaz pour raccorder fluidiquement un ventilateur mécanique fournissant un flux de gaz respiratoire contenant au moins 20%vol. d'oxygène, et un appareil de délivrance de NO fournissant un flux de mélange NO/N₂ d'une installation de fourniture de gaz à un patient de manière à y réaliser un mélange desdits flux gazeux et obtenir un mélange combiné contenant du NO, de l'oxygène (i.e. au moins 20%vol. d'O₂) et de l'azote (N₂).

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les pressions sont exprimées en bar absolus, abrévié « bar ».
- les termes « concentration », « quantité », « proportion », « dose » et « teneur » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue, ou tout autre capteur adapté.
- par « mesure de débit », on entend une valeur de débit (e.g. une valeur numérique) ou un signal représentatif d'une telle valeur de débit reflétant ou correspondant à un débit gazeux mesuré par un capteur de débit ou analogue, ou tout autre capteur adapté.
- les termes « amont » et « aval » sont utilisés par rapport au sens normal de circulation du gaz considéré, par exemple dans le sens allant du/des récipients de gaz vers l'appareil de délivrance, puis vers le patient pour le mélange NO/N₂, ou dans le sens allant du ventilateur vers le module d'injection, puis vers le patient pour le gaz respiratoire contenant de l'oxygène (i.e. >20%vol).

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de délivrance de NO.
Fig. 2 représente un mode de réalisation d'un module d'injection selon l'art antérieur.
Fig. 3 et Fig. 4 représente un mode de réalisation d'un autre module d'injection selon l'art antérieur.
Fig. 5 et Fig. 6 schématisent un mode de réalisation d'un module d'injection pour une installation selon la présente invention, telle celle de Fig. 1.
Fig. 7 est une courbe illustrant le phénomène de diffusion de gaz dans les modules d'injection des Fig. 2 à Fig. 6.
Fig. 8 schématise un mode de réalisation d'un montage expérimental utilisé lors des essais comparatifs.

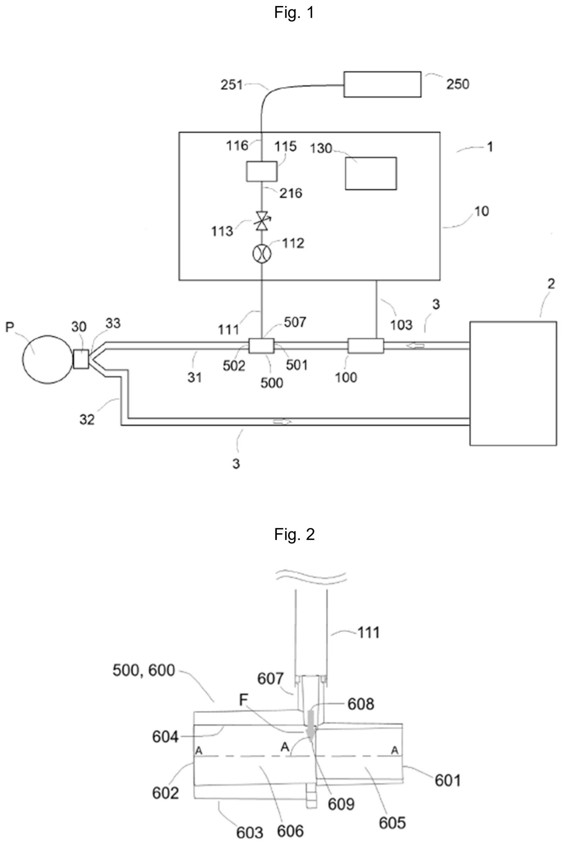

Fig. 1 schématise un mode de réalisation d'une installation de fourniture ou délivrance de gaz 1, 2 selon la présente invention comprenant un dispositif ou appareil de délivrance de NO 1 et un ventilateur mécanique 2, aussi appelé ventilateur médical, c'est-à-dire un appareil d'assistance respiratoire délivrant un gaz respiratoire non-hypoxique à base d'oxygène, alimentant un circuit patient 3, en particulier la branche inspiratoire 31 dudit circuit patient 3, de manière à obtenir un mélange gazeux combiné, aussi appelé mélange gazeux final, destiné à être administré ensuite par inhalation à un patient P à traiter, c'est-à-dire un mélange gazeux combiné contenant de l'oxygène (>20%vol., préf. >21%vol), de l'azote et une concentration désirée de NO correspondant à une posologie fixée par un médecin-anesthésiste ou analogue, typiquement entre 1 et 80 ppmv de NO.

L'appareil de délivrance de NO 1 est alimenté en NO gazeux, typiquement en mélange NO/N₂ gazeux, provenant d'une (ou plusieurs) source de NO 250 reliée fluidiquement à l'appareil de délivrance de NO 1, en particulier à une ligne haute pression 116 du dispositif de délivrance de NO 1, par une ligne d'alimentation 251, tel un conduit flexible ou analogue.

Typiquement, la source de NO 250 comprend une (ou des) bouteille de gaz sous pression renfermant un mélange de NO/N₂ contenant une concentration en NO donnée, conditionné à une pression (lorsque totalement pleine, i.e. avant soutirage) pouvant atteindre 140 à 200 bar abs, voire plus.

Selon le cas, le mélange de NO/N₂ peut comprendre une concentration ou teneur en NO : soit dite « classique », à savoir comprise entre 100 et 1000 ppmv, par exemple de l'ordre de 400 à 800 ppmv, soit dite « élevée », à savoir supérieure à 1000 ppmv, typiquement supérieure à 2000 ppmv, par exemple de 5000 à 20 000 ppmv.

L'appareil de délivrance de NO 1 permet de fournir au circuit patient 3, du NO sous forme gazeuse, typiquement le mélange gazeux NO/N₂ contenant le NO à teneur « classique » ou « élevée », alors que le ventilateur médical 2 délivre un gaz respiratoire non-hypoxique contenant au moins 20%vol. d'oxygène, de préférence au moins 21%vol. d'oxygène environ, tel que de l'air ou un mélange N₂/O₂, dans le circuit patient 3.

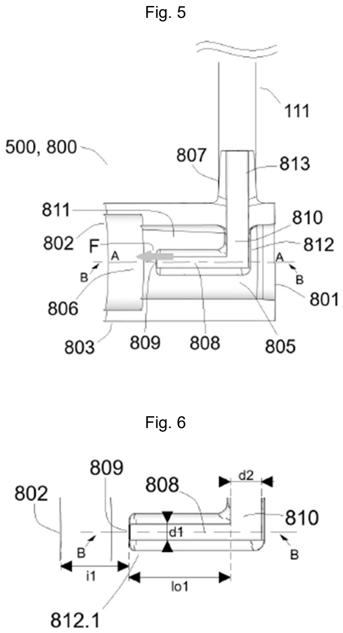

Afin de permettre d'opérer un mélange homogène entre le flux gazeux contenant le NO, c'est-à-dire le mélange NO/N₂ provenant de l'appareil de délivrance de NO 1, et le flux gazeux de gaz respiratoire contenant l'oxygène provenant du ventilateur médical 2, tel de l'air ou un mélange O₂/N₂, et ainsi obtenir le mélange combiné désiré, on agence un module d'injection de NO 500 dans la branche inspiratoire 31 du circuit patient 3, auquel vient de raccorder l'appareil de délivrance de NO 1, via une ligne d'injection 111 qui est raccordée à un canal d'injection 507 de mélange NO/N₂ du module d'injection de NO 500, comme détaillé ci-après, en particulier le module d'injection de NO 500, 800 de l'invention illustré sur Fig. 5 comme expliqué ci-après.

Plus précisément, le module d'injection de NO 500 comprend en outre :
- une entrée de gaz respiratoire, aussi appelée port amont, au niveau de laquelle vient se raccorder fluidiquement un conduit de gaz amont formant une partie amont de la branche inspiratoire 31, lequel amène le gaz respiratoire provenant du ventilateur médical,
- un canal d'injection 507 de mélange NO/N₂ fluidiquement connecté à la ligne d'injection 111 et
- une sortie de gaz combiné, aussi appelée port aval, au niveau de laquelle vient se raccorder fluidiquement un conduit de gaz aval formant une partie aval de la branche inspiratoire 31, lequel sert à recueillir le mélange gazeux combiné (i.e. NO, N₂, O₂) formé au sein du module d'injection de NO 500 et à le convoyer ensuite vers le patient.

Afin de pouvoir y raccorder fluidiquement les conduit de gaz amont et aval de la branche inspiratoire 31 du circuit patient, et/ou la ligne d'injection 111, le corps de module tubulaire 500 comprend des moyens de raccordement de conduit adaptés, tels des raccords ou analogues, par exemple à emboitement, vissés, à baïonnette ou analogues.

Les branches inspiratoire 31 et expiratoire 32 du circuit patient 3 comprennent généralement des conduits de gaz, aussi appelés canalisations, tuyaux, passages, tubulures ou similaires, par exemple des tuyaux flexibles en polymère, qui sont aptes à et configurés pour convoyer les flux gazeux.

La branche inspiratoire 31 permet ensuite d'acheminer et fournir le mélange gazeux combiné contenant le NO au patient P, pendant ses phases inspiratoires, donc de fournir une assistance respiratoire au patient P, alors que la branche expiratoire 32 permet de convoyer les gaz expirés par le patient, lors de ses phases expiratoires.

Comme visible sur Fig. 1, les branches inspiratoire 31 et expiratoire 32 sont reliées fluidiquement à une pièce de jonction 33, telle une pièce en Y ou similaire, en communication fluidique avec une interface respiratoire 30 permettant de délivrer le gaz combiné au patient P et de collecter les gaz expirés riches en CO₂ et de les acheminer jusqu'au ventilateur médical 2 avant d'être rejetés à l'atmosphère. L'interface respiratoire 30 peut être un masque respiratoire facial, une sonde d'intubation ou toute autre dispositif d'administration adapté.

Le ventilateur médical 2 est quant à lui un appareil d'assistance respiratoire classique ou analogue comprenant par exemple une (micro)soufflante motorisée, aussi appelée turbine ou compresseur, délivrant le gaz respiratoire (e.g. air ou N₂/O₂) dans le circuit patient 3 et dont le fonctionnement est contrôlé par une (ou des) carte électronique de commande ou analogue.

Le ventilateur 2 et l'appareil de délivrance de NO 1 sont l'un et l'autre alimenté électriquement par des moyens d'alimentation électrique (non montrés), tel le secteur (110/220V) et/ou une (des) batterie interne.

Par ailleurs, un capteur de débit 100 et le module d'injection de NO 500 sont agencés dans la branche inspiratoire 31 du circuit patient 3, le capteur de débit 100 étant préférablement disposé entre le module d'injection de NO 500 et le ventilateur mécanique 2.

Le capteur de débit 100 peut être un capteur massique ou à mesure de pression différentielle, de préférence un capteur de débit massique comme illustré en Fig. 1, par exemple celui de la société Sensirion^{®} référencé SFM3300-D. Le capteur de débit 100 est utilisé afin de mesurer le flux gazeux, i.e. un débit, délivré par le ventilateur médical 2 dans la partie amont de la branche inspiratoire 31. Pour ce faire, le capteur 100 peut être soit connecté électriquement, par exemple via un câble électrique 103, à l'unité de pilotage 130 de l'appareil de délivrance de NO 1, soit peut lui transmettre les mesures de débit afin qu'elles y soient traitées informatiquement.

L'unité de pilotage 130 comprend un système de traitement de données, i.e. de mesures, agencé dans la carcasse 10 de l'appareil 1, comprenant un (ou des) microprocesseur(s) agencé sur une (ou des) carte électronique et mettant en œuvre un (ou des) algorithme(s), i.e. un (ou des) programme d'ordinateur, typiquement un microcontrôleur ou analogue. En d'autres termes, l'unité de pilotage 130 est configurée pour traiter et/ou exploiter les signaux de mesure de débit ou les valeurs de débit transmises par le capteur de débit 100.

Bien entendu, l'unité de pilotage 130 peut être aussi configurée pour piloter d'autres éléments électromécaniques intégrés l'appareil de délivrance de NO 1, telles des électrovannes, ou encore pour commander des affichages sur un écran d'affichage (non montré) de l'appareil 1, tel un écran tactile.

Selon un mode de réalisation, l'unité de pilotage 130 utilise et/ou comprend (au moins) une table de correspondance préenregistrée au sein de moyens de mémorisation, telle une mémoire flash ou autre, qui permet, en fonction de la mesure de débit opérée par le capteur de débit 100, de calculer la quantité de NO devant être ajoutée au gaz circulant dans la branche inspiratoire 31 pour obtenir la posologie de NO souhaitée dans le mélange gazeux combiné devant être administré au patient P.

Autrement dit, en utilisant la (les) mesure de débit retournée par le capteur de débit 100 et la table de correspondance, l'unité de pilotage 130 peut déterminer le débit de gaz (e.g. air ou O₂/N₂) issu du ventilateur mécanique 2 et la quantité de mélange NO/N₂ devant être ajoutée, via le module d'injection de NO 500, afin d'obtenir la concentration de NO souhaitée, i.e. la posologie définie par le médecin, devant être inhalée par le patient P. La quantité de NO à fournir dépend aussi de la concentration en NO dans le mélange N₂/NO provenant de la (ou des) source de N₂/NO 250 alimentant l'appareil de délivrance de NO 1, comme expliqué ci-avant.

Plus précisément, en fonction du débit gazeux (i.e. air ou N₂/O₂) circulant dans la branche inspiratoire 31 et entrant dans le port amont 501 du module d'injection de NO 500, l'unité de pilotage 130 détermine la quantité de NO, typiquement de mélange de NO/N₂, devant être injectée par le module d'injection de NO 500 dans la branche inspiratoire 31 du circuit patient 3 afin d'obtenir la concentration de NO souhaitée dans le mélange gazeux combiné. Le mélange NO/N₂ provenant de l'appareil de délivrance de NO 1 est fourni au module d'injection 500 par une ligne d'injection 111 reliant fluidiquement l'appareil de délivrance de NO 1 au module d'injection 500, telle une conduite de gaz flexible ou analogue.

Plus précisément, le mélange NO/N₂ provenant de la (les) source de N₂/NO 250, qui est à une pression dite « haute » puisque pouvant atteindre 140 à 200 bar abs, comme expliqué ci-avant, alimente une ligne haute pression 116 du circuit interne de l'appareil 1, laquelle présente une entrée haute pression connectée fluidiquement à la (aux) source de NO 250 pour être alimentée en NO/N₂ à haute pression.

Un régulateur de pression 115 agencé sur le circuit interne de l'appareil 1, permet de réduire la pression du mélange NO/N₂ (i.e. gaz détendu) amené par la ligne haute pression 116, jusqu'à une valeur préfixée, par exemple environ 4 bar abs ou toute autre valeur désirée. Le port de sortie du régulateur de pression 115 fournit alors le mélange gazeux NO/N₂ à une ligne basse pression 216 à la pression préfixée stable, e.g. 4 bar environ.

Le gaz détendu est recueilli et acheminé par la ligne basse pression 216 du circuit interne de l'appareil 1, qui alimente ensuite la ligne d'injection 111 qui est elle-même fluidiquement raccordé au module d'injection 500, via le canal d'injection de NO 507.

Le circuit interne de l'appareil de délivrance de NO 1 comprend un (des) passage ou conduit de gaz, en particulier les lignes à haute et à basse pressions 116, 216, et est agencée dans la carcasse 10 de l'appareil 1.

Une électrovanne 113, par exemple une électrovanne proportionnelle, par exemple celle de la série VSO miniature commercialisée par la société Parker^{®}, est agencée sur le circuit interne de l'appareil 1, typiquement sur la ligne basse pression 216, et configurée pour contrôler le débit de NO/N₂ gazeux fourni à la ligne d'injection 111. Ce débit gazeux est mesuré par un capteur de débit de NO 112, préférentiellement agencé sur la ligne basse pression 216, en aval de l'électrovanne 113, comme illustré sur Fig. 1.

Le régulateur de pression 115, l'électrovanne 113, le capteur de débit de NO 112 et le circuit interne comprenant les lignes à haute et à basse pression 116, 216, sont agencés dans le boitier ou carcasse 10 du dispositif de délivrance de NO 1.

Comme déjà dit, l'extrémité aval de la ligne d'injection 111 vient se raccorder fluidiquement au canal d'injection de NO 507 du module d'injection 500 afin de permettre l'injection du mélange NO/N₂ dans le module d'injection 500 et opérant son mélange avec le flux de gaz contenant l'oxygène (e.g. air) provenant du ventilateur 2, lequel est véhiculé par la partie amont de la branche inspiratoire 31 du circuit patient 3, et entre ensuite dans le module d'injection 500 via son port amont 501, et pouvoir obtenir ainsi le mélange gazeux combiné à base de NO et d'oxygène.

Le mélange gazeux combiné obtenu ressort du module d'injection 500 par son port aval 502, et est ensuite véhiculé par la portion de branche inspiratoire 31 située en aval du module d'injection 500 jusqu'à l'interface respiratoire 30. En général, ce mélange gazeux combiné comprend principalement de l'azote (N₂), de l'oxygène (O₂) en une teneur d'au moins 20 à 21%vol., et du NO à une teneur typiquement comprise entre 1 et 80 ppmv, et éventuellement des impuretés inévitables, y compris des espèces toxiques NO₂ résultant d'une oxydation intempestive de NO. Comme expliqué ci-après, le module d'injection 500 de l'invention permet de minimiser la formation de telles espèces NO₂ toxiques, en permettant une homogénéisation rapide et efficace du mélange gazeux réalisé dans le module d'injection 500.

Selon un mode de réalisation, la branche inspiratoire 31 peut également comprendre un humidificateur (non représenté) afin d'humidifier le gaz délivré au patient P, lequel est placé en aval du module d'injection de NO 500, c'est-à-dire entre ledit module d'injection de NO 500 et l'interface respiratoire 30 fournissant le gaz au patient P.

En outre, l'installation 1, 2 comprend aussi généralement une ligne de prélèvement d'échantillons gazeux (non représentée sur Fig. 1), venant se raccorder fluidiquement à la branche inspiratoire 31, en aval du module d'injection de NO 500, c'est-à-dire entre ledit module d'injection de NO 500 et l'interface respiratoire 30 fournissant le gaz au patient P, et par ailleurs à des moyens d'analyse de l'appareil 1 (i.e. capteurs de NO, O₂, NO₂...), de manière à pouvoir prélever une partie du mélange gazeux combiné et de l'analyser au sein de l'appareil 1 afin de s'assurer que sa composition gazeuse est bien conforme à celle attenue, en particulier que sa tenir en NO correspond bien à la posologie souhaitée, que celle en oxygène est bien supérieure à au moins 20%vol, et que les impuretés néfastes NO₂ susceptibles d'être présentes restent en dessous de seuil donné, par exemple moins de quelques ppmv.

Fig. 2 est une en vue en coupe d'un mode de réalisation d'un module d'injection 500, 600 selon l'art antérieur, lequel comprend un corps tubulaire 603 de forme générale cylindrique, traversé par un passage central 608 (i.e. un lumen) présentant un axe longitudinal (AA).

Le corps tubulaire 603 du module d'injection 500, 600 comprend :
- une chambre amont 605 avec un port amont 601, aussi appelé « port ou orifice d'entrée » de gaz respiratoire, destinée à être alimentée en gaz respiratoire (O₂ >20%vol.) provenant du ventilateur 2.
- une chambre aval 606 avec un port aval 602, aussi appelé « port ou orifice de sortie », au sein de laquelle s'opère le mélange du flux de gaz respiratoire (O₂ >20%vol.) provenant du ventilateur 2 et le flux de NO/N₂ amené par le canal d'injection 607. Le mélange gazeux combiné ainsi obtenu est ensuite convoyé jusqu'au patient.
- et un canal d'injection 607, de forme cylindrique, présentant un passage interne 608 (env. 4 mm de diamètre) raccordé sensiblement à la jonction des deux chambres amont 605 et aval 606, et débouchant dans le passage central du corps tubulaire 603, c'est-à-dire approximativement au milieu du module d'injection 600.

Le flux de gaz respiratoire (i.e. air ou O₂/N₂) provenant du ventilateur 2 circule axialement (selon l'axe AA) dans le sens allant de la chambre amont 605 en direction de la chambre aval 606.

La ligne d'injection de NO 111 montrée en Fig. 1, vient se raccorder fluidiquement au canal d'injection 607 afin d'alimenter le passage interne 608 du corps tubulaire 603 en mélange NO/N₂. Le conduit interne 608 débouche au niveau d'un port d'injection 609 agencé dans la paroi interne 604, c'est-à-dire un orifice affleurant percé dans la paroi interne 604 du conduit interne 608 par lequel circule le flux de mélange NO/N₂ (flèche F).

Comme on le voit, du fait de l'orientation du conduit interne 608 sensiblement perpendiculaire à la paroi interne 604 du corps tubulaire 603, et donc aussi à l'axe AA du corps tubulaire 603, l'injection du mélange NO/N₂ dans le flux de gaz respiratoire se fait ici perpendiculairement à l'axe AA, c'est-à-dire selon un angle de 90°. Autrement dit, le flux de mélange NO/N₂ (symbolisé par la flèche F) pénètre dans le passage central 608 (lumen) du corps tubulaire 603 selon un angle A de l'ordre de 90° par rapport à l'axe longitudinal (AA) du corps tubulaire 603.

Or, un tel agencement n'est pas idéal car le port d'injection 609 se situe dans une zone de faible vélocité du gaz respiratoire provenant du ventilateur mécanique 2, au sein du module d'injection de NO 500, 600, ce qui ne permet pas une diffusion efficace du mélange N₂/NO.

Par ailleurs, l'emplacement du port d'injection 609 se faisant dans une zone de faible vélocité, il apparait que les molécules d'O₂ vont migrer dans le passage interne 608, voire dans la ligne d'injection 111, en fonction de la vélocité du gaz circulant respectivement dans la ligne d'injection 111 et dans le conduit interne 608. Les molécules d'O₂ présentes dans le conduit interne 608, voire dans la ligne d'injection 111, se retrouvent alors exposées au mélange NO/N₂ amené par la ligne d'injection 111, ce qui va favoriser la formation de NO₂, qui se retrouvera ensuite dans la chambre aval 606 du module d'injection 500, 600. Cette oxydation intempestive du NO est d'autant plus importante que la concentration en NO dans le mélange NO/N₂ amené par la ligne d'injection 111, est élevée.

Autrement dit, le module d'injection 500, 600 de Fig. 2 ne permet pas d'aboutir à un mélange rapide et homogène des flux gazeux, ni de garantir une formation limitée, de préférence aussi faible que possible, de NO₂.

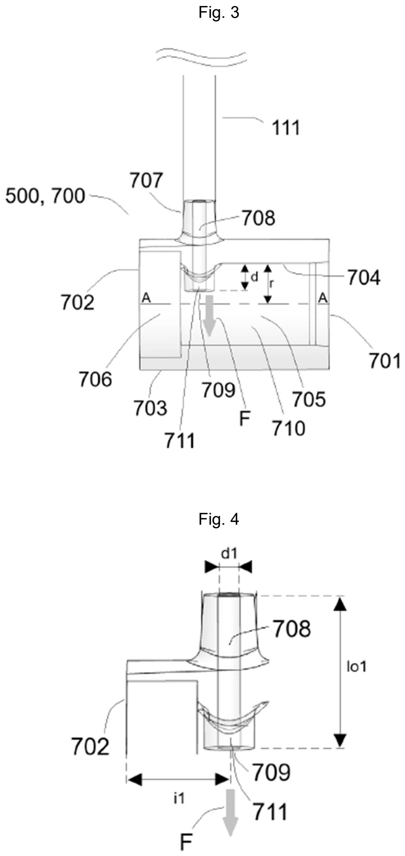

Fig. 3 et Fig. 4 sont des vues en coupe d'un autre mode de réalisation d'un module d'injection 500, 700 de l'art antérieur, tel que proposé par exemple par US10894140, lequel a une structure sensiblement analogue à celle du module d'injection 500, 600 de Fig. 2.

Ainsi, le module d'injection 500, 700 comprend là encore un corps tubulaire 703 de forme cylindrique traversé par un passage interne 710 (lumen) présentant un axe longitudinal (AA). Le passage interne comprend une chambre amont 705 comprenant un port amont 701 alimenté en gaz respiratoire provenant du ventilateur mécanique 2 ; une chambre de mélange aval 706 ; et un canal d'injection 707 de forme cylindrique et présentant un passage ou canal interne 708 de diamètre d1 et de longueur lo1. Le canal interne 708 est en communication fluidique avec la ligne d'injection 111 qui vient se raccorder audit canal d'injection 707. Le diamètre interne d1 du passage interne 708 est variable, typiquement entre 0.1 mm et 1 mm, par exemple d'environ 0.2 mm. De même, la longueur lo1 du passage interne 708 est également variable, typiquement entre 1 et 50 mm, par exemple d'environ 10mm.

Le passage ou canal interne 708 débouche sur un port d'injection 709. Le centre du port d'injection 709 du canal interne 708 est situé à une distance i1 du port aval 702 du module d'injection 700, comme illustré en Fig. 4 qui est une vue grossie d'une partie de Fig. 3.

Toutefois, ici, le port d'injection 709 n'est pas affleurant à la paroi interne 704 de l'enveloppe évidée 703, comme dans le mode de réalisation de Fig. 2, mais est déporté radialement, c'est-à-dire situé à proximité de l'axe longitudinal (AA) du corps tubulaire 703.

Plus précisément, le port d'injection 709 est porté par une expansion tubulaire 711, qui forme une buse ou analogue, se projetant radialement dans le passage interne 710 (lumen) du corps tubulaire 703 en direction de l'axe (AA). Par exemple, la distance d entre la paroi interne 704 du corps tubulaire 703 et l'extrémité libre de l'expansion tubulaire 711 portant le port d'injection 709, peut-être de l'ordre de 60% du rayon interne r du corps tubulaire 703 qui est de forme cylindrique.

Là aussi, le flux de gaz respiratoire (i.e. air ou O₂/N₂) provenant du ventilateur 2 circule axialement (selon l'axe AA) dans le sens allant de la chambre amont 605 en direction de la chambre aval 606.

De plus, on note que, comme dans le mode de réalisation de Fig. 2, l'injection du flux de NO/N₂ se fait selon une direction (quasi)perpendiculaire (flèche F). Un tel agencement vise à tirer parti de la distribution du profil de vélocité du gaz qui circule axialement (axe AA) dans le passage interne 710 (lumen) cylindrique du corps tubulaire 703 qui est maximale au centre, c'est-à-dire au niveau de l'axe AA, et en moyenne (quasi-)nulle au niveau de la paroi interne 704.

Or, l'obtention d'un mélange homogène du gaz contenant du NO et du gaz respiratoire provenant du ventilateur mécanique 2 (i.e. mélange N₂/O₂) dépend de la vélocité de ces gaz. Plus cette vélocité est élevée, plus le temps requis pour lequel le mélange de gaz est considéré homogène est faible.

Bien que le module d'injection 500, 700 des Fig. 3 et Fig. 4 conduise à de meilleurs résultats que le module d'injection 500, 600 de Fig. 2, grâce à l'utilisation d'une buse ou analogue se projetant radialement dans le passage de gaz véhiculant le flux provenant du ventilateur 2, et distribuant le mélange N₂/O₂ à une distance donnée de l'axe AA, afin de laisser le temps au mélange N₂/O₂ sortant du port d'injection 709 de se dilater avant de se mélanger avec le gaz respiratoire (i.e. air ou O₂/N₂) dans les région situées à proximité de l'axe AA, où la vélocité du flux est maximale, il est apparu, en pratique, que cette configuration n'est pas non plus idéale.

Ainsi, elle ne permet pas de supprimer totalement le phénomène de diffusion de l'O₂ dans le conduit interne 708 du fait de la perpendicularité de l'injection du gaz contenant du NO dans le gaz respiratoire issu du ventilateur mécanique 2. En effet, aux abords du port d'injection 709, de l'O₂ est toujours en capacité de migrer dans le conduit interne 708 et d'y former des espèces NO₂ toxiques au contact des molécules de NO, en particulier lorsque le mélange N₂/O₂ mis en oeuvre contient une haute concentration de NO, i.e. plus de 1000 ppmv.

Par ailleurs, l'injection transverse du mélange N₂/NO, s'éjectant à haute vélocité du port d'injection 708 va former une « plume » gazeuse au contact du mélange N₂/O₂, conduisant à concentrer une partie non-négligeable du mélange N₂/NO injecté, donc des molécules de NO, dans des zones à faible vélocité, en particulier aux abords de la paroi interne 704, en créant ainsi un déséquilibre temporaire dans la distribution du gaz dans la chambre aval 706, qui conduit à une augmentation la propension à former du NO₂ en augmentant le temps nécessaire pour obtenir un mélange gazeux homogène.

Fig. 5 et Fig. 6 schématisent un mode de réalisation d'un module d'injection 500, 800 selon la présente invention, utilisable dans une installation 1, 2, 3 de fourniture de NO, telle celle de Fig. 1 ou une installation analogue.

Ce module d'injection 500, 800 est conçu pour permettre une injection de mélange NO/N₂ ayant des teneurs en NO « classiques » (i.e. <1000 ppmv) et aussi « élevées » (i.e. > 1000 ppmv) dans le flux de gaz respiratoire provenant du ventilateur 2 et obtenir des mélanges homogènes tout en minimisant la formation de NO₂.

Le module d'injection 500, 800 a une forme générale analogue à celles des modules précédents des Fig. 2 à Fig. 4, à savoir qu'il comprend un corps tubulaire 803 de forme cylindrique, traversé par un passage interne (i.e. un lumen) et un axe longitudinal (AA). Le corps tubulaire 803 comprend une chambre amont 805 qui reçoit le gaz respiratoire (i.e. air ou mélange O₂/N₂) délivré par le ventilateur 2, via un port amont 801 (i.e. orifice d'entrée) du module d'injection 800, et une chambre de mélange aval 806 où le mélange entre le flux de NO/N₂ et le flux de gaz respiratoire issu du ventilateur mécanique 2 va s'opérer pour obtenir le mélange homogène combiné contenant le NO à la posologie souhaitée.

Le corps tubulaire 803 est préférentiellement en polymère.

La chambre de mélange aval 806 comprend un port aval 802, c'est-à-dire un orifice de sortie, par lequel le mélange gazeux combiné ressort du corps tubulaire 803 avant d'être convoyée par la partie aval de la branche inspiratoire 31 du circuit patient 3 jusqu'à l'interface respiratoire 30 alimentant le patient P, comme déjà expliqué.

Comme précédemment, le flux de gaz respiratoire (contenant >20% d'O₂) issu du ventilateur mécanique 2 circule axialement (i.e. selon l'axe AA) de la chambre amont 805 vers la chambre de mélange aval 806, c'est-à-dire dans le sens allant du port amont 801 vers le port aval 802.

Là encore, le corps tubulaire 803 du module d'injection 500, 800 comprend un canal d'injection 807 traversé par un passage interne 813 amont de diamètre d2, auquel vient se raccorder la ligne d'injection 111 amenant le mélange NO/N₂ délivré par l'appareil de délivrance de NO 1.

Comme dans le mode de réalisation précédent et illustré en Fig. 5, le port d'injection 809 alimenté notamment par le canal d'injection 807, par lequel le mélange NO/N₂ pénètre dans le passage interne 811 (lumen) du corps tubulaire 803, est porté par une expansion tubulaire 812, avantageusement constituant ou formant une buse ou analogue, se projetant dans le passage interne 811 (lumen) du corps tubulaire 803 en direction de l'axe (AA).

Toutefois, contrairement au mode de réalisation de Fig. 3 et Fig. 4, l'expansion tubulaire 812 n'est pas rectiligne mais a, selon l'invention, une forme coudée, i.e. recourbée, à savoir une forme sensiblement en « L ».

L'expansion tubulaire 812 coudée présente respectivement un premier tronçon de conduit interne 810 de diamètre d2 et un second tronçon de conduit interne 808 de diamètre d1 et de longueur lo1. Le premier tronçon de conduit interne 810 est situé entre le passage interne 813 amont et le second tronçon de conduit interne 808, comme illustré sur Fig. 5 et sur Fig. 6 qui est une vue grossie de l'expansion tubulaire 812 de Fig. 5.

Autrement dit, le passage interne 813 amont, le premier tronçon de conduit interne 810 et le second tronçon de conduit interne 808 sont en communication fluidique les uns avec les autres de sorte que le flux de NO/N₂ amené par la ligne d'injection 111 circule successivement dans ces passages/tronçons avant d'en ressortir par le port d'injection 809 qui est situé à l'extrémité libre 812.1 du second tronçon de conduit interne 808.

Le diamètre d2 du passage interne 813 amont et/ou du premier tronçon conduit interne 810 peut être compris entre 1 et 5 mm, par exemple de l'ordre de 3 mm. Dans tous les cas, il est supérieur au diamètre d1 du second tronçon de conduit interne 808 de l'expansion tubulaire 812 coudée, i.e. d2 > d1.

Comme on le voit, l'axe BB du second tronçon conduit interne 808 de l'expansion tubulaire 812 coudée, telle une buse ou analogue, aussi appelée « buse coudée » ci-après, est orientée parallèle à l'axe longitudinal AA du corps tubulaire 803 du module d'injection 500, 800, de manière à injecter le mélange NO/N₂ dans le même sens ou la même direction (i.e. selon l'axe AA) que le flux de gaz respiratoire circulant dans le passage interne 811 (lumen) du corps tubulaire 803, à savoir celui de la flèche F de Fig. 5.

Avantageusement, ils sont coaxiaux, comme illustré sur Fig. 5 et Fig. 6, c'est-à-dire que les axes AA et BB du second tronçon conduit interne 808 de la buse coudée 812 et du corps tubulaire 803 sont confondus.

Dit autrement, dans le mode de réalisation des Fig. 5 et Fig. 6, le second tronçon de conduit interne 808 de l'expansion tubulaire 812 coudée, i.e. de la buse coudée, est centré sur l'axe longitudinal AA du corps tubulaire 803, c'est-à-dire agencé de manière coaxiale à l'axe longitudinal AA, de sorte que le flux de NO sortant par le port d'injection 809 de la buse coudée 812, soit injecté au centre du corps tubulaire 803 (axe AA) et dans la même direction que le flux de gaz respiratoire circulant dans le passage interne 811 (lumen) du corps tubulaire 803, c'est-à-dire en direction de la chambre de mélange aval 806 et de son port aval 802.

Comme schématisé en Fig. 6, le second tronçon de canal interne 808 comprend un diamètre interne d1 qui peut être compris entre 0.1 et 1 mm, par exemple de l'ordre de 0.2 mm, et une longueur lo1 qui peut être comprise entre 1 et 50 mm, par exemple d'environ 10 mm.

Le second tronçon de canal interne 808 de la buse coudée 812 se termine par le port d'injection 809 qui est, lui aussi, centré sur l'axe AA et situé à une distance i1 du port aval 802 du corps tubulaire 803 du module d'injection 500, 800. La distance i1 peut être comprise entre 1 et 10 mm par exemple.

Comme on le voit, le port d'injection 809 du second tronçon de canal interne 808 de la buse coudée 812 fait face au port aval 802 du module d'injection 500, 800, c'est-à-dire qu'elles sont agencées en regard l'une de l'autre.

Dans cette configuration avantageuse selon l'invention, l'injection du flux/débit de mélange NO/N₂ se fait parallèlement et dans la même direction, de préférence coaxialement, que le flux/débit de gaz respiratoire contenant au moins 20%vol. d'oxygène (i.e. air ou mélange O₂/N₂) fourni par le ventilateur mécanique 2 circulant dans le passage interne 811 (lumen) du corps tubulaire 803, c'est-à-dire dans la direction allant du port amont 801 au port aval 802 du module d'injection 800.

Ainsi, l'expansion tubulaire ou buse coudée 812 est dirigée ou orientée de manière à délivrer le flux de mélange NO/N₂ de manière parallèle et dans le sens du flux de gaz respiratoire provenant du ventilateur 2 et circulant dans le lumen 811 du module d'injection 500, 800, de préférence coaxialement audit flux de gaz respiratoire, comme déjà expliqué.

En aval du port d'injection 809 se trouve la chambre de mélange aval 806 dans laquelle le mélange entre le débit de NO/N₂ et le débit de gaz respiratoire issu du ventilateur mécanique 2 va s'opérer pour donner le mélange homogène désiré contenant la posologie de NO souhaitée/réglée, par exemple une concentration de NO comprise entre 1 et 80 ppmv.

Selon une configuration de l'invention, le fait que les axes AA et BB soient préférentiellement coaxiaux, donc que le port d'injection 809 de la buse coudée soit situé sur l'axe AA du corps tubulaire 803 du module d'injection 500, 800, le mélange du flux de NO/N₂ avec celui de gaz respiratoire (i.e. air ou mélange O₂/N₂ avec teneur en O₂ > 20%vol.) provenant du ventilateur 1 et entrant dans le module d'injection 500, 800 par son port amont 801, se fait de manière efficace car la vélocité du gaz circulant dans le conduit cylindrique est maximale au niveau de l'axe AA, ce qui accélère le mélange des flux gazeux l'un avec l'autre.

Il en résulte une formation rapide d'un mélange homogène, alors qu'à l'inverse, la formation d'espèces NO₂ indésirables par oxydation NO avec de l'oxygène présent dans le gaz respiratoire est limitée, en particulier en comparaison avec le module d'injection 500, 700 précédent, et ce, même en présence d'une concentration (très) importante de NO dans le mélange N₂/NO, typiquement supérieure à 1000 ppmv, par exemple comprise entre 2000 et 20000 ppmv

Grâce à la configuration particulière du module d'injection 500, 800 selon l'invention de Fig. 5 et Fig. 6, la diffusion d'O₂ dans le second conduit interne 808 au travers du port d'injection 809 est réduite du fait du sens de circulation commun des flux gazeux et de l'orientation particulière de la buse coudée 812 du module d'injection 500, 800 selon l'invention. En effet, il est beaucoup plus difficile pour les molécules d'oxygène de s'opposer au mouvement directionnel général, c'est-à-dire de remonter « à contre-courant » pour migrer dans le second conduit interne 808.

Plus généralement, la configuration particulière du module d'injection 500, 800 selon l'invention de Fig. 5 et Fig. 6 fait que l'injection de gaz à haute vélocité, i.e. le flux de NO, va s'épandre plus uniformément, donc les différentes zones de la chambre aval 806 reçoivent une quantité sensiblement identique de mélange N₂/NO injecté, engendrant ainsi un équilibre distribution permettant de limiter la formation d'espèces NO₂ indésirables.

Afin de montrer l'efficacité d'un module d'injection 500, 800 selon l'invention de Fig. 5 et Fig. 6, des essais comparatifs de simulation ont été réalisés en utilisant les différents module d'injection 500 illustrés sur Fig. 2 à Fig. 6, afin d'évaluer la diffusion des molécules d'O₂ dans les conduits internes 608, 708 et 808 de ces différents modules d'injection de NO 600, 700, 800, 500.

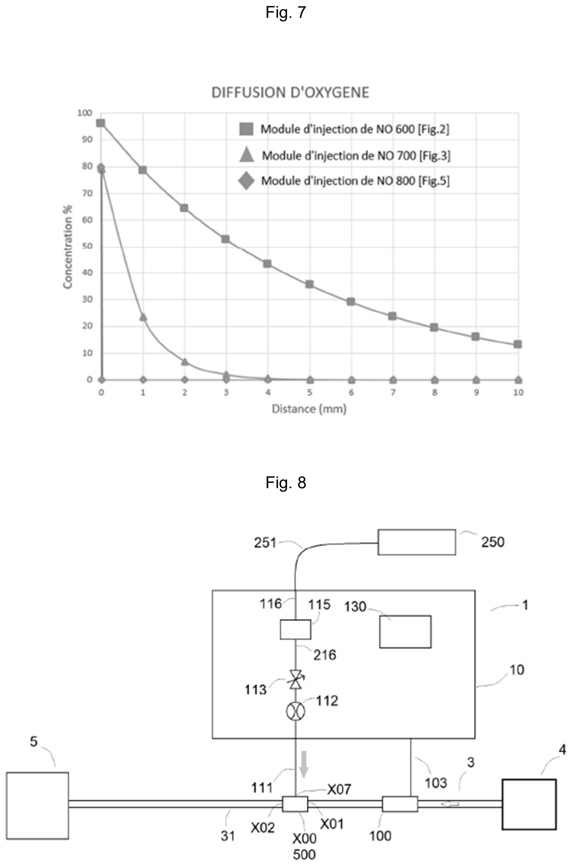

Les conditions des essais de simulation sont les suivantes :
- bouteille de N₂/NO contenant une teneur élevée en NO, à savoir ici 20 000 ppmv de NO.
- débit de 5 L/min d'oxygène pur provenant du ventilateur mécanique 2.
- posologie réglée : 80 ppmv de NO dans le gaz combiné envoyé au patient.
- le montage expérimental utilisé pour réaliser les essais est illustré en Fig. 8 et détaillé ci-après.

Les résultats obtenus ont été consignés sur Fig. 7 qui est un graphique illustrant la diffusion des molécules d'oxygène. L'axe des abscisses correspond aux distances (en mm) et celui des ordonnées donne des valeurs de concentration en O₂ (en % vol).

L'origine 0 de l'axe des abscisses correspond aux différents ports d'injection 609, 709 et 809 des modules d'injection 600, 700 et 800, c'est-à-dire que la distance de « 0 mm » est le point de référence correspondant à l'emplacement des différents ports d'injection 609, 709 et 809. Une distance positive, par exemple 2 mm, correspond à la distance séparant les ports d'injection 609, 709 et 809 d'un « point » de chaque conduit interne 608, 708 et 808, situé à 2 mm du port d'injection considéré 609, 709 et 809. De même, une autre distance positive, par exemple 5 mm, correspond à la distance séparant les ports d'injection 609, 709 et 809 d'un « point » des conduits interne 608, 708 et 808 situé à 5mm des ports d'injection 609, 709 et 809.

Il apparait que le module d'injection de NO 500, 600 de l'art antérieur de Fig. 2, dans les conditions susmentionnées (i.e. 20000 ppmv de NO), voit les molécules d'O₂ diffuser massivement dans son conduit interne 608. Au niveau de son port d'injection 609, la concentration en O₂ est proche de 100%, à une distance de 3 mm, cette concentration est de l'ordre de 50% et de 15% à une distance de 10 mm environ. Ceci est principalement dû à la faible vélocité du gaz circulant dans le conduit interne 608, du fait du diamètre relativement important du conduit interne 608, et de la méthode d'injection perpendiculaire affleurante, comme déjà expliqué.

Avec le module d'injection de NO 500, 700 de l'art antérieur des Fig. 3 et Fig. 4, dans les mêmes conditions, les molécules d'O₂ diffusent moins dans son conduit interne 708. Au niveau du port d'injection 709, la concentration en O₂ est proche de 80%. Ceci est dû à l'éjection à plus haute vitesse du mélange N₂/NO au niveau du port d'injection 709. A une distance de 1 mm, cette concentration est de l'ordre de 25% et proche de 0% à une distance de 3 mm. La haute vélocité du gaz circulant dans le conduit interne 708, de diamètre bien plus faible que le conduit interne 608 du mode de réalisation précédent, limite la progression de la diffusion de l'O₂. Toutefois, une diffusion intempestive des molécules d'O₂ se produit quand même laquelle conduit inévitablement à la formation d'espèces néfastes NO₂.

Enfin, avec un module d'injection de NO 800 selon l'invention, tel qu'illustré sur Fig. 5 et Fig. 6, mettant en oeuvre une buse coudée 812 configurée pour opérer une injection du flux de NO/N₂ centrée (i.e. coaxiale à l'axe AA) et dans le sens de circulation du gaz respiratoire issu du ventilateur mécanique 2, il n'existe aucune diffusion des molécules d'O₂ dans le conduit interne 808. En effet, une concentration en O₂ de 80% est présente uniquement au niveau du port d'injection 809 et chute à 0% à l'intérieur du conduit interne 808.

Dans ce cas, une diffusion intempestive des molécules d'O₂ ne peut pas se produire et dès lors toute formation d'espèces néfastes NO₂ sera rendue impossible.

Comme illustré en Fig. 8, le montage expérimental de mesure de performances des différents modules d'injection testés, à savoir ceux selon l'art antérieur des Fig. 2 à Fig. 4 et celui selon l'invention de Fig. 5 et Fig. 6, ayant été utilisé est globalement analogue à l'installation de Fig. 1 décrit ci-avant, en particulier pour ce qui est de l'appareil de délivrance de NO 1 et de son fonctionnement, mis à part que dans ce montage expérimental :
- le circuit patient 3 de l'installation de Fig. 1 a été simplifié par suppression de la branche expiratoire 32.
- le ventilateur mécanique 2 de Fig. 1 a été remplacé par un générateur de débit 4 d'O₂, permettant de délivrer un débit constant d'O₂ dans la branche inspiratoire 31.
- le patient a été, quant à lui, remplacé par un analyseur de gaz 5 permettant de mesure la concentration en NO₂ dans le mélange gazeux qu'il reçoit et analyse, par exemple l'analyseur référencé FTIR Multigas 2030 disponible auprès de la société MKS^{®} ou celui référencé 42iQHL disponible auprès de la société Thermo Fisher Scientific^{®}.

En Fig. 8, le module d'injection de NO X00, 500, présentant des ports amont X01 et aval X02, ainsi qu'un canal d'injection X07, a été l'un ou l'autre des modules d'injection de NO 600, 700, 800, 500 décrits auparavant, selon le test réalisé, c'est-à-dire que X peut prendre la valeur de 6, 7 ou 8 en fonction du module d'injection de NO testé.

Par ailleurs, l'analyseur de gaz 5 est situé à une distance d'environ 1 m du module d'injection X00, 500.

Les valeurs de NO₂ mesurées par l'analyseur de gaz 5 avec les différentes configurations de modules d'injection de NO 600, 700, 800, 500 décrits auparavant pour une concentration de NO de 20 000 ppmv, comme expliqué ci-dessus, sont reportées dans Tableau 1 ci-après.

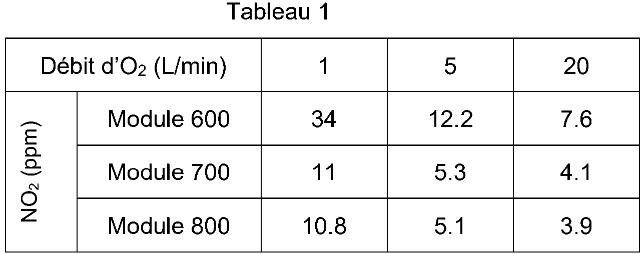

D'autres essais comparatifs, ont été réalisés avec une concentration en NO de 5000 ppm dans le mélange NO/N₂. Toutefois, dans ce cas, afin de conserver une vélocité de gaz similaire circulant dans les conduits internes 708, 808 des modules d'injection de NO 700, 800, le diamètre d1 desdits conduits internes 708, 808 a été porté à 0.4 mm environ.

Dans ces conditions, les valeurs de NO₂ mesurées par l'analyseur de gaz 5 pour les différentes configurations de modules d'injection de NO 600, 700, 800, 500 décrits auparavant pour une concentration de NO de 5000 ppmv, sont reportées dans Tableau 2 ci-après.

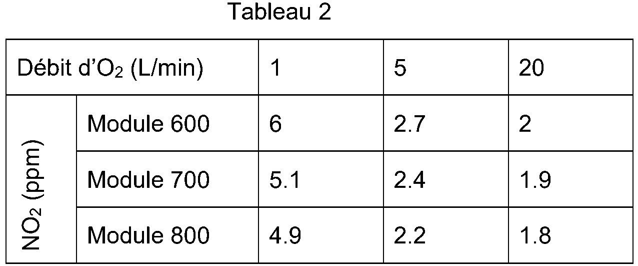

Ces essais montrent que, quelle que soit la concentration en NO (i.e. 5000 ou 20000 ppmv), les modules d'injection de NO 700, 800 de Fig. 3 à Fig. 6 permettent de réduire significativement la concentration en NO₂ formée par rapport au module d'injection de NO 600 de Fig. 2. Ceci démontre l'intérêt à utiliser une expansion tubulaire, telle une buse d'injection ou analogue, se projetant dans le passage interne (lumen) du corps tubulaire.

Toutefois, la configuration du module d'injection de NO 500, 800 selon l'invention mettant en œuvre une expansion tubulaire courbée 812 orientée pour permettre d'opérer une injection du débit de N₂/NO parallèlement, et dans le même sens que le mélange gazeux provenant du ventilateur mécanique 2 (ici de l'O₂ provenant générateur de débit 4 d'O₂ lors des essais) permet de réduire encore plus la formation de NO₂ par rapport au module d'injection de NO 500, 700 de l'art antérieur selon les Fig. 3 et Fig. 4 qui propose d'utiliser une buse rectiligne orientée vers l'axe AA, du fait d'une moindre diffusion de l'O₂ dans le conduit interne 808 du module d'injection de NO 500, 800 selon l'invention, ainsi qu'une homogénéisation plus rapide des mélanges gazeux N₂/NO et par exemple N₂/O₂ ou air dans sa chambre aval 806.

D'une façon générale, l'installation de l'invention est particulièrement bien adaptée au traitement de personnes, i.e. un patients humains (i.e. adultes, enfants, adolescents ou nouveau-nés), souffrant d'hypertension pulmonaire et/ou d'hypoxie, engendrant des vasoconstrictions pulmonaires ou analogues, résultant d'une (ou des) pathologie ou autres troubles pulmonaires typiquement de type PPHN (hypertension pulmonaire persistante du nouveau-né) ou SDRA (syndrome de détresse respiratoire aigüe), ou encore pouvant se produire dans le cadre d'une opération de chirurgie cardiaque avec mise sous circulation sanguine extracorporelle (CEC).

## Revendications

1. Installation de fourniture de gaz (1, 2, 3) comprenant un module d'injection de gaz (500, 800), auquel sont raccordés :
- un ventilateur mécanique (2) fournissant un flux de gaz respiratoire contenant au moins 20%vol. d'oxygène, et
- un appareil de délivrance de NO (1) fournissant un flux de mélange gazeux NO/N₂,
ledit module d'injection de gaz (500, 800) comprenant un corps de module tubulaire (803) traversé par un passage interne (811) comprenant un axe longitudinal (AA),
ledit corps de module tubulaire (803) comprenant :
- un port amont (801) par lequel le flux de gaz respiratoire peut entrer dans le passage interne (811),
- un port aval (802) par lequel un flux gazeux combiné peut ressortir du passage interne (811),
- un conduit d'injection (807) externe venant se raccorder fluidiquement au corps de module tubulaire (803), et comprenant un passage interne (813) pour véhiculer le flux de mélange gazeux de NO/N₂, et
- une expansion tubulaire (812) formant buse d'injection agencée dans le passage interne (811) du corps tubulaire (803) et comprenant un passage de buse interne (808) en communication fluidique avec le passage interne (813) du conduit d'injection (807), ladite expansion tubulaire (812) comprenant en outre un port d'injection (809) agencé à une extrémité libre (812.1) de ladite expansion tubulaire (812), ledit port d'injection (809) débouchant dans le passage interne (811) du corps de module tubulaire (803),
**caractérisée en ce que** l'expansion tubulaire (812) du corps de module tubulaire (803) du module d'injection (500, 800) a une forme générale courbe ou coudée, et comprend au moins un tronçon portant le port d'injection (809), agencé parallèlement à l'axe longitudinal (AA) et dirigé en direction du port aval (802).

2. Installation selon la revendication 1, **caractérisée en ce que** le passage interne (811) du corps de module tubulaire (803) du module d'injection (500, 800) comprend une chambre amont (805) comprenant le port amont (801) et/ou une chambre de mélange aval (806) comprenant le port aval (802).

3. Installation selon la revendication 1, **caractérisée en ce que** l'expansion tubulaire (812) du corps de module tubulaire (803) du module d'injection (500, 800) a une forme générale en « L ».

4. Installation selon la revendication 1, **caractérisée en ce que** le tronçon (808) portant le port d'injection (809) de l'expansion tubulaire (812) du corps de module tubulaire (803) du module d'injection (500, 800) est agencé coaxialement à l'axe longitudinal (AA) du passage interne (811) du corps de module tubulaire (803).

5. Installation selon l'une des revendications 1, 3 ou 4, **caractérisée en ce que** l'expansion tubulaire (812) du corps de module tubulaire (803) du module d'injection (500, 800) comprend un premier tronçon de conduit (810) et un second tronçon conduit (808), où :
- le second tronçon conduit (808) comprend le port d'injection (809) et l'extrémité libre (812.1) de ladite expansion tubulaire (812), et
- le premier tronçon de conduit (810) est agencé entre le second tronçon conduit (808) et le passage interne (813) du conduit d'injection (807) et en communication fluidique avec le second tronçon conduit (808) et le passage interne (813).

6. Installation selon la revendication 1, **caractérisée en ce que** le conduit d'injection (807) externe du corps de module tubulaire (803) du module d'injection (500, 800) vient se raccorder à la paroi périphérique tubulaire du corps de module tubulaire (803).

7. Installation selon la revendication 1, **caractérisée en ce que** le corps de module tubulaire (803) du module d'injection (500, 800) est configuré, au niveau du port amont (801) et du port aval (802), pour permettre un raccordement fluidique de conduits de gaz formant au moins une partie de la branche inspiratoire (31) du circuit patient (3).

8. Installation selon la revendication 1, **caractérisée en ce que** l'appareil de délivrance de NO (1) est relié fluidiquement et alimenté en un mélange NO/N₂ par un ou plusieurs récipients de gaz (250) contenant un mélange NO/N₂.

9. Installation selon l'une des revendications 1 ou 8, **caractérisée en ce que** l'appareil de délivrance de NO (1) est alimenté en un mélange NO/N₂ contenant entre 100 et 20 000 ppmv de NO, le reste étant de l'azote (N₂).

10. Installation selon la revendication 1, **caractérisée en ce que** le module d'injection (500, 800) est agencé dans une branche inspiratoire (31) d'un circuit patient (3), ladite branche inspiratoire (31) étant alimentée en gaz respiratoire contenant au moins 20%vol. d'oxygène par le ventilateur mécanique (2).

11. Installation selon la revendication 1, **caractérisée en ce que** l'expansion tubulaire (812) du corps de module tubulaire (803) forme une buse courbée.

12. Installation selon la revendication 1, **caractérisée en ce que** l'expansion tubulaire (812) du module d'injection de gaz (500, 800) est orientée pour opérer une injection du mélange NO/N₂, dans le passage interne (811) d'axe longitudinal (AA) dans la même direction que le flux de gaz respiratoire contenant au moins 20%vol. d'oxygène, circulant dans le passage interne (811).

13. Installation selon la revendication 1, **caractérisée en ce que** l'appareil de délivrance de NO (1) comprend des moyens de pilotage, un circuit de gaz interne, des moyens à vanne agencées sur le circuit de gaz interne pour contrôler le flux de NO/N₂ dans le circuit de gaz interne, et au moins une sortie de NO pour fournir le flux gazeux de NO/N₂.

14. Installation selon la revendication 1, **caractérisée en ce qu'**un capteur de débit est agencé dans le circuit patient (3) entre le ventilateur (2) et ledit module d'injection de gaz (500, 800).

15. Installation selon la revendication 1, **caractérisée en ce que** le ventilateur mécanique (2) est configuré pour fournir un flux de gaz respiratoire contenant au moins 20%vol. d'oxygène choisi parmi de l'air ou un mélange O₂/N₂.
